# EUROPEAN PATENT APPLICATION

(11) **EP 4 822 024 A1**
(43) Date of publication of application: **14.10.2026**
(21) Application number: 25738476.8
(22) Date of filing: 08.01.2025
(51) Int. Cl.: C12N 15/12, C12N 7/01, A61K 48/00, A61P 3/06

(54) **CODON-OPTIMIZED LDLR GENE AND USE THEREOF**

(30) Priority: 08.01.2024 CN 202410026314
(71) Applicant: Suzhou NGGT Biotechnology Co., Ltd., Suzhou, Jiangsu 215125 (CN)
(72) Inventor: WEN, Shengmei, Suzhou, Jiangsu 215125 (CN); GUO, Minjun, Suzhou, Jiangsu 215125 (CN); ZHANG, Hai, Suzhou, Jiangsu 215125 (CN); TAI, Zhenzhen, Suzhou, Jiangsu 215125 (CN); LIU, Yiting, Suzhou, Jiangsu 215125 (CN); JIANG, Lixin, Suzhou, Jiangsu 215125 (CN)
(74) Representative: karo IP
(86) International application number: PCT/CN2025/071379
(87) International publication number: WO 2025/148947

(57) **Abstract**

The present disclosure provides a nucleic acid molecule, a transgenic expression cassette, a recombinant adeno-associated virus (rAAV), and uses thereof. In the present disclosure, a nucleic acid molecule encoding a human low-density lipoprotein receptor is assembled together with expression control elements that facilitate expression in liver cells to obtain a transgenic expression cassette, which is then used to prepare a recombinant adeno-associated virus (rAAV). The resulting rAAV can effectively reduce LDL-C and TC levels in subjects at a relatively low dose over a long term, showing prospects in the treatment of familial hypercholesterolemia.

## Description

### Technical Field

The present disclosure belongs to the technieilcal field of gene therapy, and particularly relates to a nucleic acid molecule, a transgene expression cassette, a recombinant adeno-associated virus and uses thereof, as well as a medicament.

### Background Art

Cholesterol is an essential cellular component, and maintaining cholesterol homeostasis is critical for normal physiological function. Elevated plasma cholesterol levels are associated with various pathological conditions, most notably coronary heart disease, in which high cholesterol levels lead to foam cell formation and arterial plaque accumulation, potentially causing heart attacks or strokes. Regulation of cellular cholesterol metabolism and plasma cholesterol levels depends on low-density lipoprotein receptor (LDLR)-mediated uptake of LDL into specific cells. LDL is the major carrier of cholesterol in blood, and accounts for more than 60% of total plasma cholesterol. LDL is taken up by the liver and extrahepatic tissues via receptor-mediated endocytosis triggered by the apoB-100-LDLR interaction. The internalised LDL particles are transported to lysosomes, where they are degraded into free cholesterol and amino acids. In humans, the liver is the most important organ for the catabolism of LDL-C (low-density lipoprotein cholesterol) and for LDL-C receptor activity.

Hypercholesterolaemia is one of the most significant independent risk factors for atherosclerosis and cardiovascular diseases, and it interacts with numerous genetic and environmental factors. Familial hypercholesterolaemia (FH) is the most common inherited form of primary hypercholesterolaemia, its pathogenesis is primarily due to genetic mutations of the low-density lipoprotein (LDL) receptor, leading to reduced function or loss of function, slowed clearance of LDL from the plasma, and elevated cholesterol levels. This results in premature cardiovascular and cerebrovascular disease, and often accompanied by xanthomas of the skin and tendons.

FH is an autosomal dominant genetic disorder; the prevalence of heterozygous mutations in the LDLR gene is estimated to be 1 in 200 to 1 in 500, and the prevalence of homozygous mutations is approximately 1 in 100,000. FH is one of the most severe common inherited metabolic disorders, despite its high prevalence, it still remains underdiagnosed and undertreated. It has been reported that over 50% of patients over 60 years of age with myocardial infarction having heterozygous LDLR mutations (Wiegman A, Gidding SS, Watts GF, et al. Familial hypercholesterolaemia in children and adolescents: gaining decades of life by optimising detection and treatment [J]. Eur Heart J. 2015, 36(36):2425-2437.), and most of patients with homozygous mutations do not receive effective treatment and die before the age of 30. LDL-C levels in heterozygous patients are more than double the normal level (approximately 5 mmol/L), while homozygous patients had levels that were more than four times the normal level (>13 mmol/L) (Nohara A, Tada H, Ogura M, et al. Homozygous Familial Hypercholesterolemia[J]. J Atheroscler Thromb. 2021, 28(7):665-678.), (Defesche JC, Gidding SS, Harada-Shiba M, et al. Familial hypercholesterolaemia[J]. Nat Rev Dis Primers. 2017.). An LDL-C level of 5 mmol/L or higher indicates a high risk of severe familial hypercholesterolaemia; the ideal LDL-C level should be maintained below 2.5 mmol/L, and a reduction of more than 50% in LDL-C for FH patients is the clinical treatment target (Wierzbicki A S, Humphries S E, Minhas R. Familial hypercholesterolaemia: Summary of NICE guidance[J]. BMJ Clinical Research, 2008.).

Currently, the main marketed drugs available for lipid-lowering treatment in patients with FH include statins and PCSK9 inhibitors, such as inclisiran, evolocumab (REPATHA) and alirocumab (PRALUENT). The mechanism of action of statins is to upregulate LDLR expression, their therapeutic efficacy is significantly better in adolescent and paediatric FH patients than in adult patients, furthermore, in adolescent and children HeFH patients, most of the patients can achieve treatment targets. In adult patients, even at the maximum recommended dose, plasma LDL-C levels were reduced by only 10-25% in the majority of patients, and fewer than 30% achieved the adult treatment target. In patients with homozygous familial hypercholesterolaemia (HoFH), the average reduction in LDL-C levels ranges between 14-31%, failing to reach treatment targets. Whereas PCSK9 inhibitors act by inhibiting the degradation of the LDLR, they are effective in treating heterozygous familial hypercholesterolaemia (HeFH) but are less effective in treating HoFH.

Patients with HoFH caused by LDLR mutations respond poorly to drugs such as statins and PCSK9 inhibitors designed to increase LDLR expression, as they either lack LDLR expression or express the LDLR mutants that are inactive or have reduced activity. These patients with HoFH who are unresponsive to the main lipid-lowering drugs (statins and PCSK9 inhibitors) can only undergo non-pharmacological interventions such as lipoprotein plasma exchange, liver transplantation, and gene technology. These measures place a significant physical and financial burden on patients, and there is an urgent need for novel therapeutic approaches. Regenxbio, a US company, has developed RGX-501, a liver-directed gene therapy based on AAV. This therapy has been granted Orphan Drug Designation by the FDA, and a clinical trial has been conducted to evaluate its efficacy in patients with HoFH (clinical trial number NCT02651675). However, this clinical trial revealed that the low-dose group (2.5×10¹²GC/kg) did not achieve clinically significant changes in LDL-C levels, furthermore, there were cases requiring cortisol supplementation due to elevated transaminases.

Furthermore, no other clinical studies using optimized LDLR sequences for the treatment of FH have been reported. There is a need to develop a safer and more effective LDLR gene therapy.

### Summary of the Invention

In order to solve the aforementioned problems existing in the prior art, the present disclosure provides a nucleic acid molecule, a transgenic expression cassette, a recombinant adeno-associated virus, and use thereof. The present disclosure delivers a nucleic acid sequence encoding the human low-density lipoprotein receptor (hLDLR) protein to hepatocytes via an AAV vector, wherein the nucleic acid sequence has been obtained through codon optimization and its expression is regulated by a liver-specific expression control element. Mouse animal experiments show that the recombinant adeno-associated virus of the present disclosure can significantly reduce LDL-C and TC levels in mice at low doses over a long period.

In a first aspect, the present disclosure provides a nucleic acid molecule encoding a human low-density lipoprotein receptor.

The nucleic acid molecule comprises the nucleotide sequence as shown in any one of SEQ ID NO: 2-18.

In some preferred embodiments, the nucleotide sequence is as shown in SEQ ID NO: 3, SEQ ID NO: 4, SEQ ID NO: 5, SEQ ID NO: 11, SEQ ID NO: 12, or SEQ ID NO: 16. In some more preferred embodiments, the nucleotide sequence is as shown in SEQ ID NO: 3 or SEQ ID NO: 11. Even more preferably, the nucleotide sequence is as shown in SEQ ID NO: 11.

In the present disclosure, the nucleic acid sequence encoding the human low-density lipoprotein receptor is codon-optimized, resulting in a higher expression level of the human low-density lipoprotein receptor compared to the uncodon-optimized nucleic acid sequence encoding the wild-type human low-density lipoprotein receptor.

In a second aspect, the present disclosure provides a transgenic expression cassette.

The transgenic expression cassette includes the nucleic acid molecule described above.

In some embodiments, the transgenic expression cassette further includes a promoter, an intron, and a terminator, wherein the 3' end of the intron is operably linked to the 5' end of the nucleic acid molecule, the 3' end of the promoter is operably linked to the 5' end of the intron, and the 3' end of the nucleic acid molecule is operably linked to the 5' end of the terminator.

In some embodiments, the promoter is selected from the group consisting of a SerpinA1 gene promoter, a SerpinG1 gene promoter, an ALB gene promoter, a SerpinA1 gene promoter, an HLP gene promoter, or an LP gene promoter. In some preferred embodiments, the promoter is the SerpinA1 gene promoter. In some more preferred embodiments, the SerpinA1 gene promoter is the human α1 antitrypsin promoter, and comprises the nucleotide sequence as shown in SEQ ID NO: 21; and/or,

The intron is a truncated SerpinA1 intron, and in some preferred embodiments, the truncated SerpinA1 intron comprises the nucleotide sequence as shown in SEQ ID NO: 22; and/or,

The terminator is selected from the group consisting of BGH polyA, hGH polyA, or GH polyA. In some preferred embodiments, the terminator is BGH polyA. In some more preferred embodiments, the BGH polyA is a polyadenylation signal of bovine growth hormone, and comprises the nucleotide sequence as shown in SEQ ID NO: 23.

In some embodiments, the transgene expression cassette further includes a 5'ITR, a 3'ITR, and an enhancer, wherein the 5'ITR is located at the 5' end of the transgene expression cassette, the 3'ITR is located at the 3' end of the transgene expression cassette, and the 3' end of the enhancer is operably linked to the 5' end of the promoter. The 5'ITR comprises the nucleotide sequence as shown in SEQ ID NO: 19, and the 3'ITR comprises the nucleotide sequence as shown in SEQ ID NO: 24. In some preferred embodiments, the enhancer is an ApoE HCR enhancer. In some more preferred embodiments, the ApoE HCR enhancer is the hepatocyte control region of human apolipoprotein E, and comprises the nucleotide sequence as shown in SEQ ID NO: 20.

In some embodiments, the transgenic expression cassette further includes a Kozak sequence located between the intron and the nucleic acid molecule described above, and the nucleotide sequence of the Kozak sequence is gccacc.

In this disclosure, the liver specific expression control elements are selected and assembled with the nucleic acid sequence encoding human low density lipoprotein receptor to obtain a transgenic expression cassette, which can efficiently express human low density lipoprotein receptor protein.

Furthermore, the transgenic expression cassette comprises the nucleotide sequence as shown in SEQ ID NO: 32.

In a third aspect, the present disclosure provides a recombinant adeno-associated virus.

The recombinant adeno-associated virus includes an AAV capsid and the nucleic acid molecule or transgenic expression cassette described above. In some preferred embodiments, the AAV is selected from the group consisting of AAV1, AAV2, AAV3, AAV4, AAV5, AAV6, AAV7, AAV8, AAV9 or rhAAV10. In some more preferred embodiments, the AAV is AAV8.

In this disclosure, an expression cassette containing a human low-density lipoprotein receptor can be efficiently delivered to liver cells by selecting an AAV capsid that tends to the liver and constructing a recombinant adeno-associated virus for gene delivery together with a transgenic expression cassette.

In a fourth aspect, the present disclosure provides a composition.

The composition includes the nucleic acid molecules described above, the transgenic expression cassette described above, or the recombinant adeno-associated virus described above.

In some preferred embodiments, the composition is a pharmaceutical composition, and in some more preferred embodiments, the pharmaceutical composition further includes a pharmaceutically acceptable carrier and/or excipient.

In a fifth aspect, the present disclosure provides the use of the nucleic acid molecule described above, the transgenic expression cassette described above, the recombinant adeno-associated virus described above, or the composition described above in the preparation of a medicament for treating hypercholesterolemia.

In some preferred embodiments, the hypercholesterolemia is familial hypercholesterolemia.

In some preferred embodiments, the familial hypercholesterolemia includes homozygous familial hypercholesterolemia and heterozygous familial hypercholesterolemia.

In the disclosure, the pharmaceutical composition can be used to transduce cells in vitro or to transduce animals in vivo (e.g., mice or humans) to treat familial hypercholesterolemia.

Compared with the prior art, the present disclosure has the following beneficial effects:
In the present disclosure, the nucleic acid sequence encoding the human low-density lipoprotein receptor was codon-optimized, resulting in higher expression levels of the human low-density lipoprotein receptor, as well as enhanced lipoprotein uptake and lipid-lowering activity, both in vitro and in vivo. Furthermore, a transgenic expression cassette is obtained by combining and assembling the nucleic acid molecule encoding the human low-density lipoprotein receptor with expression control elements that facilitate expression in liver cells. This cassette is then used to prepare recombinant adeno-associated virus (rAAV). The resulting recombinant adeno-associated virus can reduce LDL-C and TC levels in subjects for a long time at a lower dose, and has a good application prospect in the treatment of familial hypercholesterolemia.

### BRIEF DESCRIPTION OF THE DRAWINGS

Figure 1 illustrates a schematic structural diagram of the hLDLR expression cassette structure constructed in Eample 1 of the present disclosure;
Figure 2 illustrates the detection results of the expression of codon-optimized hLDLR opt in HepG2 cells in Example 2 of the present disclosure; wherein (A) is the Western-Blot detection results; (B) is the quantitative results of grayscale analysis;
Figure 3 illustrates the lipid uptake level of codon-optimized hLDLR opt in Huh7 cells in Example 3 of the present disclosure;
Figure 4 illustrates the detection results of LDL-C level and hLDLR concentration in the serum of high-fat fed LDLR^{-/-} mice treated with the AAV8 recombinant virus carrying the codon-optimized hLDLR gene in Example 4 of the present disclosure; wherein (A) is the LDL-C level in the serum of the mice at weeks 1, 2 and 3; (B) is the LDL-C level in the serum of the mice at week 3.
Figure 5 illustrates the dose-response results of the AAV8 recombinant virus carrying the hLDLR gene in reducing LDL-C and TC in the serum of high-fat fed LDLR^{-/-} mice in Example 5 of the present disclosure; wherein (A) is the Western-Blot detection result of hLDLR protein expression in mouse liver after 5 weeks of administration; (B) is the detection result of LDL-C and TC level in serum of the mice per week within 5 weeks after administration.
Figure 6 illustrates the results demonstrating that the NGGT-006 recombinant virus continuously reduces serum LDL-C and TC levels in high-fat fed LDLR^{-/-} mice in the example 6 of the disclosure; wherein (A) is the detection result of LDL-C level; and (B) is the detection result of TC level.
Figure 7 illustrates the results that the NGGT-006 recombinant virus continuously and effectively reduces the levels of LDL-C and TC in the blood and decreases plaque deposition in high-fat fed LDLR^{-/-} mouse models in Example 7 of the present disclosure; wherein, (A) is the detection results of LDL-C and TC levels; (B) is the size and distribution of atherosclerotic plaques after staining with Oil Red O; and (C) is the result of aortic deposition area.
Figure 8 illustrates the comparative analysis results of the effects of the NGGT-006 recombinant virus and TBG.PI.hLDLRco.RGB AAV8 recombinant virus on reducing LDL-C and TC levels in high-fat fed LDLR -/- mouse models in Example 8 of the present disclosure; wherein, (A) is the detection result of LDL-C level; (B) is the detection result of TC level .
Figure 9 illustrates the results demonstrating that the NGGT-006 recombinant virus consistently and stably reduces LDL-C and TC levels in high-fat fed LDLR-/- golden hamsters in Example 9 of the present disclosure;
Figure 10 illustrates the results demonstrating that the NGGT-006 recombinant virus consistently and stably reduces LDL-C and TC levels in natural-diet fed LDLR^{+/-} golden hamsters in Example 10 of the present disclosure.

### DETAILED DESCRIPTION

The technical solution of the present disclosure will now be clearly and completely described with reference to specific embodiments. Obviously, the described embodiments are merely some, not all, of the embodiments of the present disclosure. All other embodiments obtained by those skilled in the art based on the embodiments of the present disclosure without creative effort fall within the scope of protection of the present disclosure.

Experimental methods not specified in the examples are generally performed under conventional conditions and as described in the manual, or as recommended by the manufacturer. Unless otherwise specified, the general equipment, materials, reagents, etc. used are commercially available.

The experimental animals used in this disclosure are as follows:
LDLR^{-/-} mice were purchased from Changzhou Cavens Laboratory Animal Co., Ltd., strain number: C000114. The serum cholesterol (TC) levels of normal mice are approximately 2.07~2.58mmol/L, wherein the low-density lipoprotein cholesterol (LDL-C) level accounts for approximately 20% of the total cholesterol (TC) level. However, the serum cholesterol (TC) levels of the homozygous LDLR^{-/-} mice are approximately 5.17~10.34 mmol /L. When high-fat diet was fed, serum cholesterol (TC) levels significantly increased (approximately 50mmol/L). This mouse model is currently recognized as an animal model for studying lipid metabolism and L DLR gene therapy.

LDLR^{-/-} and LDLR^{+/-} golden hamsters were donated from the Cardiovascular Institute of Peking University. The serum cholesterol (TC) levels of normal golden hamsters are approximately 3.46~3.82mmol/L, wherein the low-density lipoprotein cholesterol (LDL-C) level accounts for about 22% of the total cholesterol (TC) level. The serum cholesterol (TC) levels of these LDLR^{-/-} and LDLR^{+/-} golden hamsters are 13.86~27.77mmol/L and 11.5~15.22mmol/L, respectively. After feeding with high-fat and high-cholesterol diet for 4 weeks, the serum cholesterol (TC) level increased significantly (about 60 mmol/L). Compared to the mouse, the golden hamster is capable of expressing cholesteryl ester transfer protein (CETP), more susceptible to atherosclerosis (AS), and the predilection site is coronary artery, which is more similar to human lipid metabolism and AS lesions, so it is a more ideal model for lipid metabolism research.

In this disclosure, the tail vein injection method in mice is as follows:
Mice were placed in small animal cages and irradiated with an infrared heating lamp until the tail blood vessels were dilated. The mice were then removed from the cages and placed on a tail vein imaging device for rats and mice with the tails exposed. The tail veins were repeatedly wiped with alcohol cotton balls to fully expand the tail vein blood vessels. The drug was injected using a disposable 1mL sterile syringe (B. Braun, # 1.0 ml U-40 type 7). The injection was slowly pushed into the mouse through the tail vein, the injection time should be great than 10 seconds, and the injection speed should be slow and uniform. After the injection, the needle was gently withdrawn, and clean and dry cotton balls were used to apply pressure to the injection site to stop bleeding.

The method for injecting the external jugular vein of golden hamsters is as follows:
The neck skin of golden hamsters was depilated with an animal razor, and the hamsters were anesthetized with isoflurane gas. Under a dissecting microscope, the clavicle of the golden hamsters were located. Above the clavicle, near the right side of the trachea, the skin was incised with surgical scissors. Surgical forceps were used to retract the skin, subcutaneous muscle, fat, and other tissues, avoiding bleeding. Under the dissecting microscope, the external jugular vein was located, and other tissues outside the blood vessel were separated as much as possible. A disposable 1mL sterile syringe (B. Braun, # 1.0 ml U-40 type 7) was used for the injection. After aspirating the appropriate virus sample, the syringe needle was moderately bent using surgical forceps. The upper end of the blood vessel was gently lifted with surgical forceps, keeping the vessel straight. The virus sample was slowly injected into the blood vessel using the syringe. After the injection, the syringe needle was retained in the blood vessel. A piece of appropriately sized autologous fat was cut in advance. When the needle was withdrawn, the injection site was immediately covered and occluded using a small piece of autologous adipose tissue to facilitate hemostasis. After confirming that there was no bleeding from the injection site, the surgical wound was sutured. The muscle layer and skin layer were sutured using surgical needles and sutures, separately. The surgical site was disinfected with povidone-iodine, and appropriate postoperative care was performed.

The method of obtaining mice/golden hamsters serum is as follows:
The day before the collection, the mice/golden hamsters were fasted overnight but allowed free access to water. On the second day, the mice/golden hamsters were anesthetized with isoflurane gas anesthesia, and 200µL of blood was collected from the orbital venous plexus. The whole blood was kept at room temperature for more than 1 hour, and centrifuged at 5000 rpm/min for 15 minutes to obtain the serum. The serum was stored at -20°C for one week, and at -80°Cfor long-term storage.

The method for staining of the aortic arch in mice with Oil Red O is as follows:
Mice were placed in a small animal anesthesia machine for anaesthesia and euthanasia. They were fixed under a dissecting microscope, and the abdomen was disinfected with alcohol. The mice were incised using surgical instruments to expose the abdominal and thoracic cavities, and the intact blood vessels to be dissected was located, including the aortic arch. The mice were subjected to cardiac perfusion with 1×PBS. Perfusion was stopped once the outflowing perfusion fluid no longer appeared bloody. Using ophthalmic scissors and forceps, the blood vessels of the mice were completely stripped, removing as much surrounding fat as possible. The stripped blood vessels were cut open down the middle so that they could be laid flat on a glass slide, the vascular branches near the aortic arch were also cut open individually. The cut-open intact blood vessels were then placed in 1× PBS solution.

Immediately before use, 6 parts of saturated oil red O dye solution and 4 parts of distilled water were thoroughly mixed, and then filtered by qualitative filter paper to obtain oil red O working solution, and the working solution was protected from light. The blood vessels were removed from 1× PBS and spread on a glass slide, with their plaque side facing upward. The flattened vessels were then stained with the freshly prepared Oil Red O working solution for 7 minutes, and the light was kept away throughout the process. The stained blood vessels were rinsed with 75% alcohol solution until there was no color at the plaque-free part of the blood vessels, and fixed in 4% paraformaldehyde solution. The fixed stained blood vessels were tiled on the slides for scanning and photographing. During the photographing process, the slides were dripped with 1× PBS in time to keep the blood vessels moist.

The method for measuring LDL-C and TC levels in serum is as follows:
The concentration of TC in serum was determined using a total cholesterol (TC/TCH) assay kit (catalog number: A111-1-1, Nanjing Jiancheng Biotechnology Institute). The concentration of low-density lipoprotein cholesterol (LDL-C) in serum was determined using a low-density lipoprotein cholesterol (LDL-C) assay kit (catalog number: A113-1-1, Nanjing Jiancheng Biotechnology Institute).

### Example 1: Construction of adeno-associated virus vector and production, purification, and quantification of the virus.

### 1.1 Codon Optimization of the Nucleic Acid Sequence Encoding the Human Low-Density Lipoprotein Receptor

The nucleic acid sequence encoding the wild-type human low-density lipoprotein receptor (hLDLR WT) (GeneID: 3949) was found in the NCBI database (https://www.ncbi.nlm.nih.gov/), and its specific sequence is as shown in SEQ ID NO: 1. The NCBI accession number for hLDLR WT is NP_000518.1.

The nucleic acid sequence encoding wild-type human low-density lipoprotein receptor (WT hLDLR) was codon-optimized to obtain codon-optimized nucleic acid sequences, denoted as hLDLR opt1~17, and their nucleotide sequences are as shown in SEQ ID NO: 2~18 respectively.

### 1.2 Construction of hLDLR expression cassette

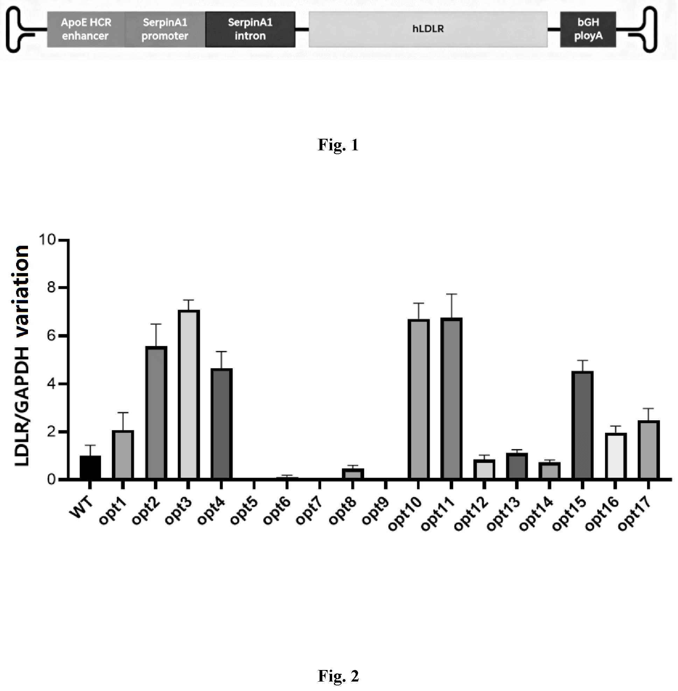

The structure of the hLDLR expression cassette is as shown in Figure 1. From the 5 ' end to the 3' end, the hLDLR expression cassette contains sequentially a 5' ITR, an ApoE HCR enhancer, a SerpinA1 promoter, a truncated SerpinA1 intron, a Kozak sequence, the target gene (hLDLR WT or codon-optimized hLDLR opt1~17), a BGH polyA, and a 3 ' ITR. Within the hLDLR expression cassette:

### 5 ' ITR is as shown in SEQ ID NO: 19;

The ApoE HCR enhancer is the hepatocyte control region of human apolipoprotein E, and the nucleotide sequence of which is as shown in SEQ ID NO: 20.

The SerpinA1 promoter is a humanα1 antitrypsin promoter, and the nucleotide sequence of which is as shown in SEQ ID NO: 21.

The SerpinA1 intron is a truncated α1 antitrypsin intron with 261 bp in length, and the nucleotide sequence of which is as shown in SEQ ID NO: 22;

The Kozak sequence was inserted before the hLDLR gene sequence, and the nucleotide sequence of which is: gccacc;

BGH polyA is the bovine growth hormone polyadenylation signal, and the nucleotide sequence of which is as shown in SEQ ID NO: 23;

### 3 ' ITR is as shown in SEQ ID NO: 24;

For example, the expression cassette containing wild-type human low-density lipoprotein receptor (hLDLR WT) consists of, in order from the 5' end to the 3' end, a 5' ITR, an ApoE HCR enhancer, a SerpinA1 promoter, a truncated SerpinA1 intron, a Kozak sequence, the target gene (hLDLR WT), a BGH polyA, and a 3' ITR, the nucleotide sequence of which is as shown in SEQ ID NO: 25.

The expression cassette containing the codon-optimized human low-density lipoprotein receptor (hLDLR opt1~17) consists of, in order from the 5' end to the 3' end, a 5' ITR, an ApoE HCR enhancer, a SerpinA1 promoter, a truncated SerpinA1 intron, a Kozak sequence, the target gene (codon-optimized hLDLR opt1~17), a BGH polyA, and a 3' ITR. The nucleotide sequences of the hLDLR opt1~17 expression cassettes were derived from the hLDLR-WT expression cassette by replacing the nucleotide sequence of hLDLR WT with one of the nucleotide sequences of hLDLR opt1~17 (for example, the hLDLR opt1 expression cassette is derived from the hLDLR-WT expression cassette by replacing the nucleotide sequence of hLDLR WT with the nucleotide sequence of hLDLR opt1). Since the nucleotide sequences of hLDLR opt1~17 have been described above, the nucleotide sequences of the hLDLR-opt1~17 expression cassettes are well-defined.

### 1.3 Production and purification of adeno-associated virus

The expression cassettes from step 1.2 were constructed into shuttle plasmids (the plasmid containing hLDLR WT was denoted as pAAV8-ATT-LDLR-WT, and the nucleotide sequence of which is as shown in SEQ ID NO: 26; the plasmids containing codon-optimized hLDLR opt1~17 were denoted as pAAV8-ATT-LDLR-opt1~17). pAAV8-ATT-LDLR-opt1~17 were obtained based on pAAV8-ATT-LDLR-WT by replacing the nucleotide sequence of hLDLR WT with one of the nucleotide sequences of hLDLR opt1~17 (taking pAAV8-ATT-LDLR-opt1 as an example, it was obtained based on the nucleotide sequence of pAAV8-ATT-LDLR-WT by replacing the nucleotide sequence of hLDLR WT with the nucleotide sequence of hLDLR opt1). Since the nucleotide sequences of hLDLR opt1~17 have been described above, the nucleotide sequences of pAAV8-ATT-LDLR-opt1~17 were well-defined. Using a three-plasmid system consisting of shuttle plasmid pAAV8-ATT-LDLR-WT or pAAV8-ATT-LDLR-opt1~17, pRepCap plasmid carrying the AAV vector *rep* and *cap* genes(the nucleotide sequence of which is as shown in SEQ ID NO: 27 and was synthesized by Genewiz), and helper plasmid pHelper (the nucleotide sequence of which is as shown in SEQ ID NO: 28, and was synthesized by Genewiz), HEK293 cells were co-transfected with PEI as transfection reagent for the recombinant packaging of AAV viral vectors. The cells were harvested at 48 hours after the transfection, and the harvested solution was purified to obtain the recombinant AAV viral vectors with a certain purity, designated as AAV8-ATT-LDLR-WT and AAV8-ATT-LDLR-opt1~17, respectively.

The purification method is as follows:
Firstly, the harvested solution was pretreated: HEK293 cells were fully lysed to release the AAV viral vector in the cells, and nuclease was added to digest the free nucleic acid. After digestion, the solution was centrifuged to remove cell debris, and the supernatant was filtered through a 0.22µm filter membrane, and the filtrate was used for affinity chromatography.

Affinity chromatography was used to capture AAV viral vectors from the harvested solution through specific ligand-protein adsorption, and the majority of process-related impurities were removed to achieve the concentration and purification. The collected eluate was mixed, neutralized with neutralization buffer, and stored in a sterile stock bottle as the loading solution for anion exchange chromatography.

The full and empty AAV viruses were separated by anion chromatography using the isoelectric point difference of different components, and residual impurities were also removed. The eluate was collected into a new sterile storage bottle and then the buffer was replaced with a stable buffer by ultrafiltration concentration method. At the same time, the virus titer was concentrated to approximately 1×10¹³vg /mL, and finally sterilized, filtered and aliquoted for later use.

### 1.4 Titer quantification of AAV Viral Vectors

Primers and probes were designed using the common sequence BGH PolyA of the AAV viral vector, and their sequences are as shown below:
F primer sequence: 5' - tgccttccttgaccctgg-3 ' (SEQ ID NO: 29);
R primer sequence: 5' - actcagacaatgcgatgcaa-3 ' (SEQ ID NO: 30);
Probe sequence: 5' - cactcccactgtcctttcctaata-3 ' (SEQ ID NO: 31);

Then, Q-PCR detection was performed to quantify the AAV viral vector.

In the process of genomic titer detection, the first step was to establish a standard curve. Positive standard plasmids were diluted with sample diluent to 2×10⁷, 2×10⁶, 2×10⁵, 2×10⁴, 2×10³, and 2×10²copies/µL to serve as templates for the standard curve. The standard curve needs to be controlled for linearity and amplification efficiency, generally, an R² value >0.99 was required, with an amplification efficiency between 90% and 110%. Then, the purified AAV viral vector (rAAV) samples were diluted and subjected to Q-PCR detection, ensuring the Ct value for the sample detection was within the standard curve range. The Ct value of the sample was then interpolated onto the standard curve to calculate the rAAV sample genomic titer for quantifying the product level.

### Example 2: In vitro expression assay of codon-optimized hLDLR opt

In this example, the in vitro expression level of codon-optimized hLDLR opt was evaluated in the HepG2 hepatocyte line (purchased from the Cell Bank of the Chinese Academy of Sciences, catalog number: TCHU72).

The shuttle plasmids pAAV8-ATT-LDLR-WT and pAAV8-ATT-LDLR-opt1~17 from step 1.3 of Example 1 were introduced into HepG2 cells to transient transfect and express the target gene by liposome transfection. Then, the expression level of the target protein in the HepG2 cell lysate was determine by Western blot analysis. The results are as shown in Figure 2.

Wherein the method for transfecting plasmids with liposomes was as follows:
HepG2 human liver cancer cells were digested and seeded into 24-well plates at a density of 1×10⁵cells/well. Simultaneously, Lipo3K/DNA transfection complex was added for plasmid transfection experiment. Firstly, Lipofectamine 3000 and P3000 (Thermo, L3000015) transfection reagent were pre-mixed with the plasmid; Then, pAAV8-ATT-LDLR-WT or pAAV8-ATT-LDLR-opt1~17 shuttle plasmid at 240ng/well, 3000 K at 360 nL/well, and Lipo 3000 at 360 nL/well (Thermo, L3000015) were added to the 24-well HepG2 cells, and incubated in a CO₂ incubator for 48 h.

The Western blot analysis method was as follows:
100µL of RIPA lysis buffer (Beyotime, P0013B) containing 1×SDS loading buffer was added to each well of a 24-well plate inoculated with cultured cells, which were lysed on ice for 5 min, then the cell protein lysate was collected and incubated on ice for 30 min, centrifuged and the protein supernatant was collected. Protein loading buffer was added to the protein sample and denatured at 95°C for 5min. 20µL of protein was loaded onto SDS-PAGE for electrophoresis, then transfered to a PVDF membrane and incubated with Anti-LDLR (abcam, ab52818), Anti-Flag (abcam, ab49763), and Anti-GAPDH (TransGen, HC301) antibodies. Image analysis was performed using a ChemiDoc Touch Imaging System (Bio-Rad) 20, and grayscale analysis was performed using ImageJ software.

As shown in Figure 2, the expression level of a portion of the codon-optimized hLDLR opt gene is significantly higher than that of the wild-type hLDLR WT gene. Therefore, in this disclosure, through careful design and extensive experiments by the inventors, the nucleotide sequences of hLDLR opt2 (as shown in SEQ ID NO: 3), hLDLR opt3 (as shown in SEQ ID NO: 4), hLDLR opt4 (as shown in SEQ ID NO: 5), hLDLR opt10 (as shown in SEQ ID NO: 11), hLDLR opt11 (as shown in SEQ ID NO: 12), and hLDLR opt15 (as shown in SEQ ID NO: 16) with high expression levels were obtained.

### Example 3: Assay of lipid uptake activity of codon-optimized hLDLR opt in hepatocytes

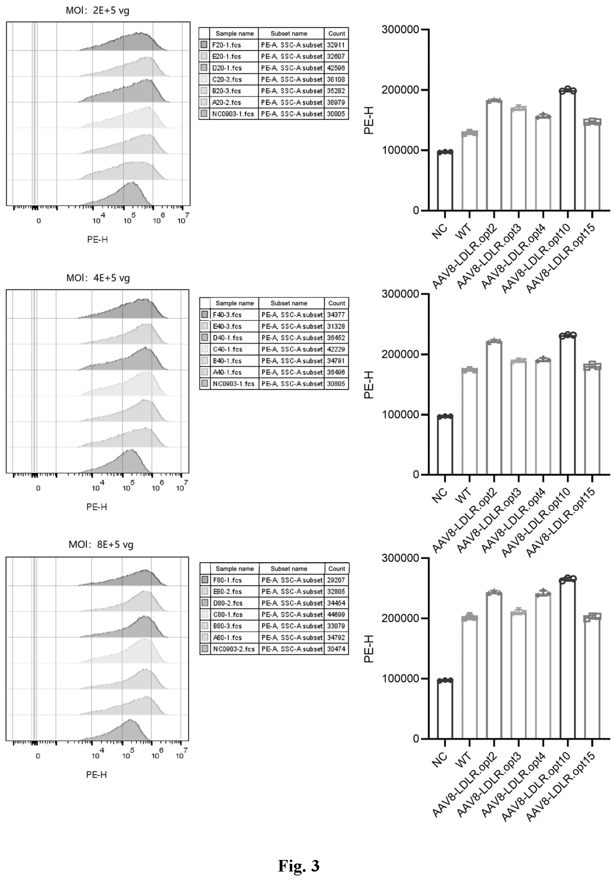

The recombinant viruses AAV8-hLDLR-WT, opt2, opt3, opt4, opt10, and opt15 prepared in Example 1 were inoculated into human hepatocellular carcinoma cells huh7 (purchased from the Cell Bank of the Chinese Academy of Sciences, catalog number: TCHU182) in 24-well plates at different MOI doses of 2E+5, 4E+5, and 8E+5, respectively on the day before the infection, 300 µL of Dil-LDL (YEASEN, 20614ES76) working solution with a concentration of 10µg/mL was added. The fluorescence signal intensity was tested using the PE channel (excitation light 549 nm, emission light 565 nm) in FACS. The results are as shown in Figure 3.

It can be seen from Fig.3 that under the experimental conditions of three different infection doses, the lipid uptake capacity of hepatocytes increased with the increasing infection dose, indicating that the codon-optimized gene significantly enhanced the lipid uptake capacity of hepatocytes compared to the wild-type gene. Furthermore, although the expression levels of opt 10 and opt 2 in transfected cells were lower than opt 3, they were more effective in enhancing the lipid uptake capacity of transfected cells. Among them, the recombinant virus AAV8-hLDLR-opt10 exhibited the highest lipid uptake capacity, followed by AAV8-hLDLR-opt2.

### Example 4: Study on the efficacy of AAV8 recombinant viruses carrying the codon-optimized hLDLR gene in reducing LDL-C levels in high-fat-fed LDLR^{-/-} mouse models

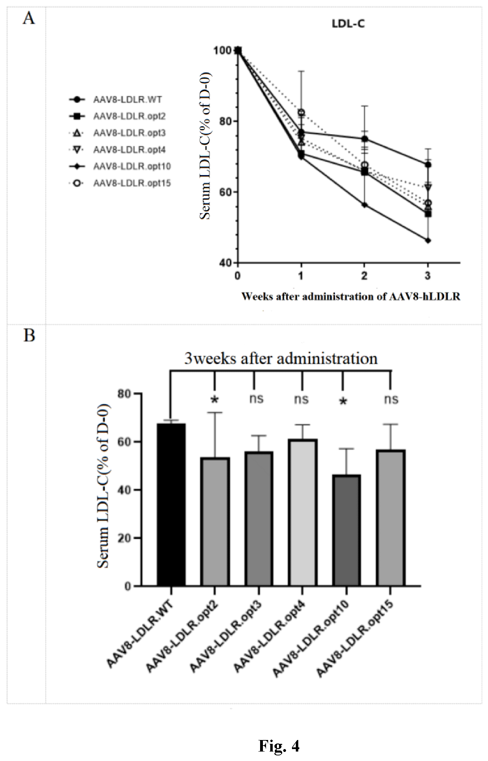

Six-week-old LDLR^{-/-} male mice were fed with high-fat, and high-cholesterol diet (2% fat+0.15% cholesterol, Ruide Biotechnology, D12079B) for eight weeks, and the LDL-C level in the serum of the mice was tested. When the LDL-C level was 10 mM, the mice were randomly divided into six groups of five mice each. Each group of mice was administered a single tail vein injection of AAV8-hLDLR-WT, opt2, opt3, opt4, opt10, and opt15 recombinant viruses (prepared in Example 1) at a dose of 8E+10 vg/kg. The LDL-C level in the serum of the mice was measured at weeks 1, 2, and 3, and the concentration of hLDLR in the serum of the mice was measured at week 3. The results are as shown in Figure 4.

As can be seen from Figures 4A and 4B, after treatment, the LDL-C level in serum of the mice gradually decreased, and the recombinant viruses AAV8-hLDLR-opt2 and AAV8-hLDLR-opt10 statistically significantly reduced LDL -C in mice.

Based on the results in Figures 4A and 4B, the recombinant virus AAV8-hLDLR-opt10 with better lipid-lowering effect and higher hLDLR concentration in the serum of the mice was designated NGGT-006.

As can be seen from Examples 1-4, firstly, in the disclosure, the nucleic acid sequence encoding the human low-density lipoprotein receptor (hLDLR) was codon-optimized, thereby generating multiple codon-optimized nucleotide sequences (designated hLDLR opt1 through (hLDLR opt1~17). These sequences were then combined with expression control elements that facilitate expression in hepatocytes to prepare shuttle plasmids, and these plasmids were transfected into hepatocyte host cells via liposomes. The protein expression levels of the codon-optimized nucleotide sequences were compared and screened by Western blotting (WB), and the sequences with significantly increased expression levels ( hLDLR opt2, hLDLR opt3, hLDLR opt4, hLDLR opt10, hLDLR opt11 and hLDLR opt15 nucleotide sequences ) were obtained. Furthermore, the sequences with higher expression levels were evaluated for their in vitro lipid uptake activity, and hLDLR-opt2 and hLDLR-opt10 were obtained as the sequences with higher lipid uptake capacity. Finally, the candidate optimized genes were packaged into AAV8 serotype recombinant viruses in HEK293 cells using a three-plasmid system. The lipid-lowering activity of the candidate sequences was evaluated in high-fat-fed LDLR^{-/-} mouse models, and the sequences (hLDLR-opt2 and hLDLR-opt10) that demonstrated superior LDL-C-lowering effects at the same viral dose were obtained.

For example, the recombinant virus NGGT-006 was employed in subsequent animal experiments for the evaluation of long-term lipid-lowering efficacy.

### Example 5: Dose response of AAV8 recombinant virus carrying the hLDLR gene in reducing LDL-C and TC in high-fat-fed LDLR^{-/-}mouse models

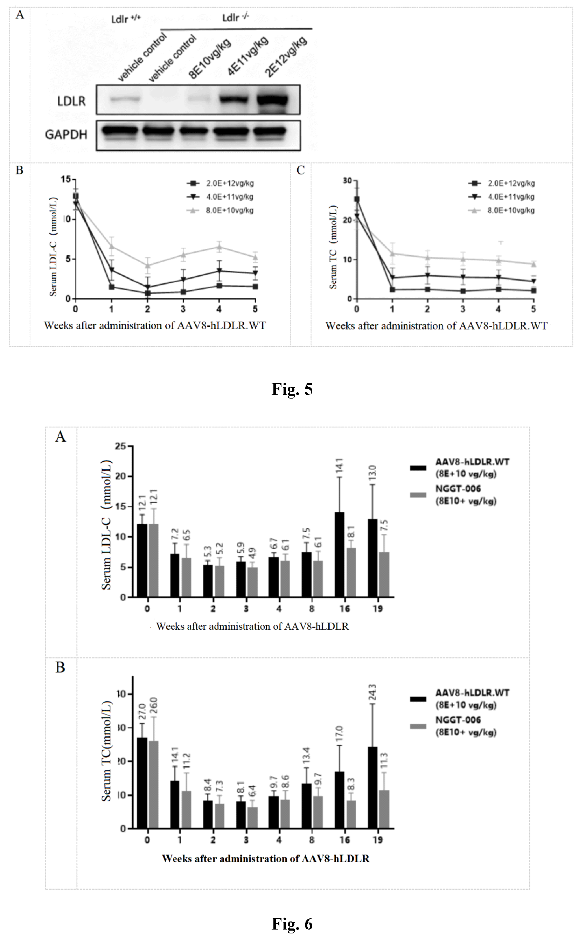

Six-week-old LDLR^{-/-} male mice were fed with high-fat, and high-cholesterol diet (2% fat + 0.15% cholesterol, Ruide Biotechnology, D12079B) for 8 weeks, and the LDL-C level in the serum of the mice was tested. When the LDL-C level was 10mM, the mice were randomly divided into three groups of five mice each. They were given a single tail vein injection of AAV8-hLDLR-WT virus (prepared in Example 1) at different gradually increasing doses of 8E +10vg/kg, 4E+11vg/kg, and 2E12 vg/kg, respectively. The LDL-C and TC levels in the serum of the mice were detected at weeks 1, 2, 3, 4, and 5, and the protein expression level of hLDLR in the mice liver was detected at week 5. The results are as shown in Figure 5.

As shown in Figure 5A, the protein expression of hLDLR in the liver increased significantly with increasing administration dose. As shown in Figure 5B, the serum LDL-C and TC levels showed a graded decrease with increasing administration dose. When the lowest dose of 8E+10vg/kg was administered, the LDL-C and TC levels decreased by approximately 50%, while when the high dose of 2E+12 vg/kg was administered, the LDL-C and TC levels decreased by more than 90%. The results indicate that the recombinant virus carrying the wild-type hLDLR gene in AAV8 can significantly reduce the serum LDL-C and TC levels in FH mice in a dose-dependent manner.

### Example 6: NGGT-006 recombinant virus continuously reduces LDL-C and TC levels in high-fat-fed LDLR ^{-/-} mouse models

Six-week-old LDLR^{-/-} mice of single-sex male were fed with high-fat, and high-cholesterol diet (2% fat + 0.15% cholesterol, Ruide Biotechnology, D12079B) for 7 weeks to establish hypercholesterolemia (FH) models. After the LDL-C level in the serum of the mice reached 10 mM, the mice were randomly divided into two groups of 5 mice each. The mice were given a single tail vein injection of AAV8-hLDLR-WT virusand NGGT-006 virus (prepared in Example 1) at a dose of 8E+10vg/kg, respectively. The LDL-C and TC levels in the serum of the mice were measured at weeks 1, 2, 3, 4, 8, 16, and 19 after injection. The results are as shown in Figure 6.

As shown in Figure 6A, both the wild-type hLDLR gene delivered by AAV8 and NGGT-006 significantly reduced LDL-C by more than 40% from week 1, and the effect of reducing LDL-C was sustained for more than 12 weeks. However, at this dosage and in the high-fat-fed FH model, the LDL-C level of the wild-type hLDLR gene delivered by AAV8 increased to above 10 mM at week 16, while NGGT-006 continuously maintained the LDL-C level below 10 mM until week 19. As shown in Figure 6B, both the wild-type hLDLR gene delivered by AAV8 and NGGT-006 significantly reduced TC by more than 45% from week 1. However, starting from week 4, the TC level in mice with the wild-type hLDLR gene delivered by AAV8 increased to above 10 mM, while NGGT-006 continuously maintained the TC level around 10 mM until week 19. The results show that the NGGT-006 optimized in this disclosure not only has a higher expression ability than the wild-type hLDLR gene, but also has better pharmacokinetics in vivo, and could maintain a therapeutically significant lipid-lowering effect over a longer time window, thus having the potential to significantly reduce the frequency of administration and presenting greater value for clinical development.

### Example 7: NGGT-006 recombinant virus consistently and effectively reduced serum LDL-C and TC levels and decreased plaque deposition in high-fat-fed LDLR^{-/-} mouse models

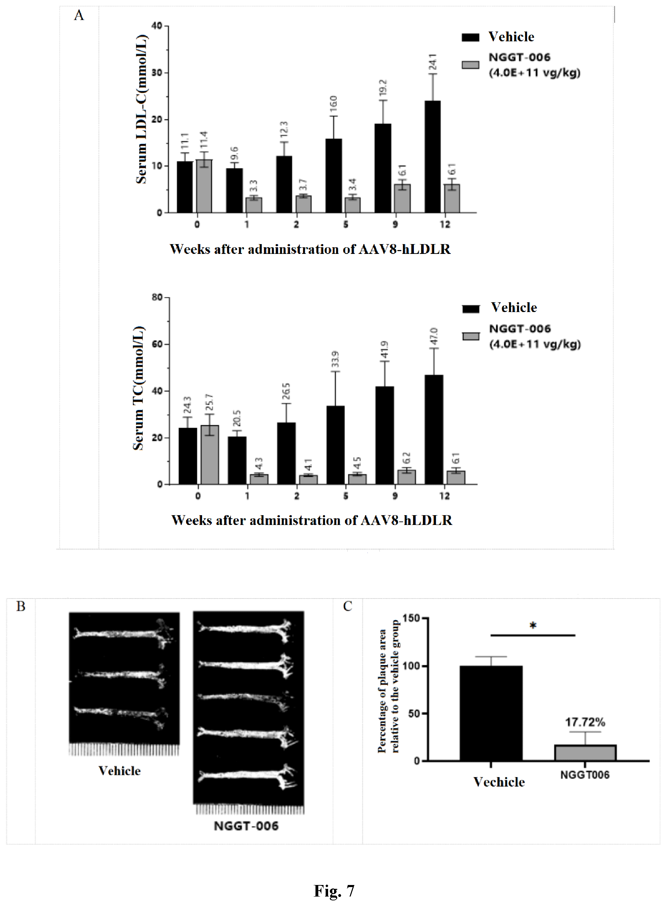

Six-week-old LDLR^{-/-} mice of single-sex male were fed with high-fat, and high-cholesterol diet (2% fat + 0.15% cholesterol, Ruide Biotechnology, D12079B) for 7 weeks to establish hypercholesterolemia (FH) models. After the LDL-C level in the serum of the mice reached 10mM, the mice were randomly divided into two groups of 5 mice each. One group was given a single tail vein injection of NGGT-006 virus (prepared in Example 1) at a dose of 4E+11vg/kg, and the other group was given an equal volume of vehicle control. The LDL-C and TC levels in the serum of the mice were measured at weeks 1, 2, 5, 9, and 12 after injection. At the end of the experiment at week 12, the mice were sacrificed and the entire aorta was isolated. Oil Red O staining was used to observe the size and distribution of atherosclerotic plaques. The results are as shown in Figure 7.

As shown in Figure 7A, compared with the vehicle control group, the drug-treated group significantly reduced the LDL-C and TC levels in the serum of the mice and the LDL-C and TC levels were maintained at low levels for at least 12 weeks. As can be seen from Figures 7B and 7C, Oil Red O staining revealed that FH mice given 4E+11 vg/kg of NGGT-006 and fed with high-fat, and high-cholesterol diet for 19 weeks (7 weeks of high-fat diet before grouping + 12 weeks of observation after drug administration) developed only mild atherosclerosis. In contrast, mice in the vehicle control group fed with high-fat, and high-cholesterol diet for 19 weeks, exhibited severe atherosclerosis throughout the aorta and aortic sinus. Furthermore, the area of atherosclerotic plaques in the drug-treated group was significantly lower than that in the vehicle group. These results indicated that the drug-treated group significantly reduced LDL-C and TC levels in the serum of the mice, and at 3 months post-drug administration, the area of atherosclerotic plaques in the drug-treated group was reduced by approximately 80% compared with the control group.

### Example 8: Comparative analysis of the effects of NGGT-006 and TBG.PI.hLDLRco.RGB recombinant virus on reducing serum LDL-C and TC in high-fat-fed LDLR^{-/-} mouse models

In Example 7, NGGT-006 significantly reduced the LDL-C and TC levels in the serum of mice at a dose of 4×10¹¹ vg/kg, and also reduced the area of atherosclerotic plaques in mice, demonstrating a significant lipid-lowering effect in the FH mouse models.

Meanwhile, based on existing public reports, the lipid-lowering effect of the sequence TBG.PI.hLDLRco.RGB (disclosed in US Patent US20190002917 A1), which was currently undergoing clinical research (NCT02651675), was used as a control group to compare the difference in lipid-lowering effect between it and NGGT-006 disclosed in this disclosure in LDLR^{-/-} FH mouse models.

Six-week-old LDLR^{-/-} mice of single-sex male were fed with high-fat, and high-cholesterol diet (2% fat+0.15% cholesterol, Ruide Biotechnology, D12079B) for 7 weeks to establish hypercholesterolemia (FH)models. After the LDL-C level in the serum of the mice reached 10 mM, the mice were randomly divided into two groups of 5 mice each. The mice were given a single tail vein injection of NGGT-006 (prepared in Example 1) and TBG.PI.hLDLRco.RGB AAV8 recombinant virus at a dose of 8E+10vg/kg, respectively. The LDL-C and TC levels in the serum of the mice were measured at weeks 1, 2, 3, 4, and 8 after injection.

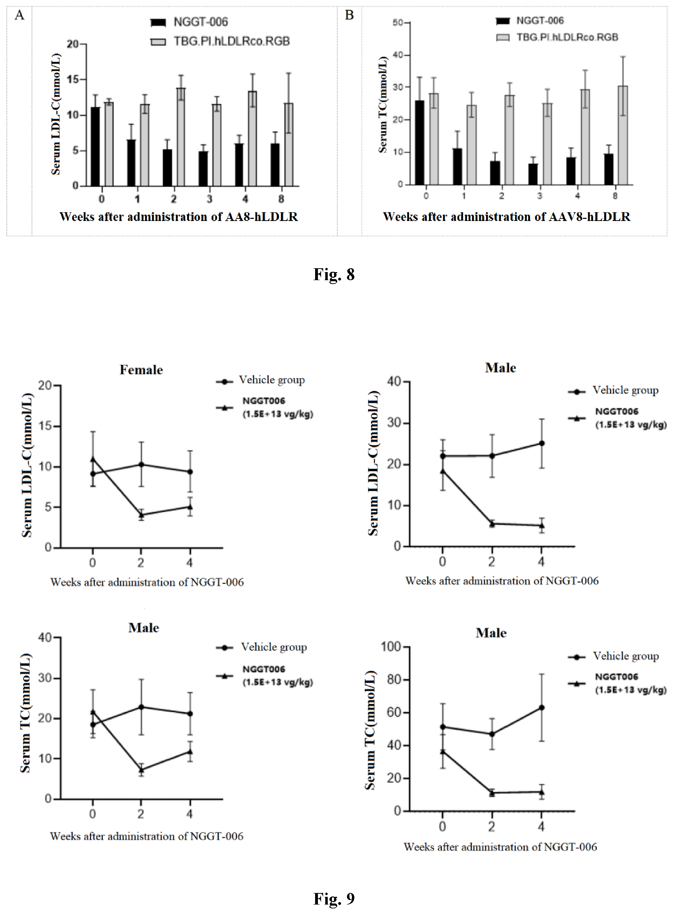

As shown in Figure 8, at a dose of 8E10 vg/kg, NGGT-006 significantly reduced the LDL-C and TC levels in the serum, while the same dose of TBG.PI.hLDLRco.RGB AAV8 failed to reduce the LDL-C and TC levels. As disclosed in US20190002917A1, TBG.PI.hLDLRco.RGB did not show any reduction in TC level at an administered viral dose of 1.5 E+11 GC/kg (same vg/kg), while a marked reduction in TC level was observed only at a dose of 5.0×10¹¹ GC/kg. Therefore, the NGGT-006 disclosed herein can achieve the effect of reducing the TC and LDL-C levels at a significantly lower dose (approximately 6-fold lower). The reduced effective dose also suggests that the NGGT-006 disclosed herein has a higher safety potential.

### Example 9: NGGT-006 recombinant virus consistently and stably reduces reduced the LDL-C and TC levels in high-fat-fed LDLR^{-/-} golden hamsters

Six-week-old LDLR^{-/-} golden hamsters were fed with high-fat, and high-cholesterol diet ( 1.5 % fat +0.5% cholesterol, Ruide Biotechnology, D06050505) to establish hypercholesterolemia (FH) models. After the LDL-C level in the serum reached 10 mM, the hamsters were randomly divided into male experimental group, male control group, female experimental group, and female control group, with five in each group. The experimental group received a single intravenous injection of 1.5E+13vg/kg NGGT-006 virus (prepared in Example 1), while the control group received the same volume of vehicle. The levels of LDL-C and TC in serum were measured at different time points after injection, and the results are as shown in Figure 9.

As shown in Figure 9, NGGT-006 significantly reduced the LDL-C and TC levels in serum of both female and male LDLR^{-/-} homozygotes, and this lipid-lowering effect lasted for at least 4 weeks. This suggests that gene therapy based on the optimized sequence disclosed in this disclosure can achieve long-term, stable reduction of LDL-C and TC with only a single dose.

### Example 10: NGGT-006 recombinant virus effectively reduced the LDL-C and TC levels in LDLR^{+/-} golden hamsters on a natural diet

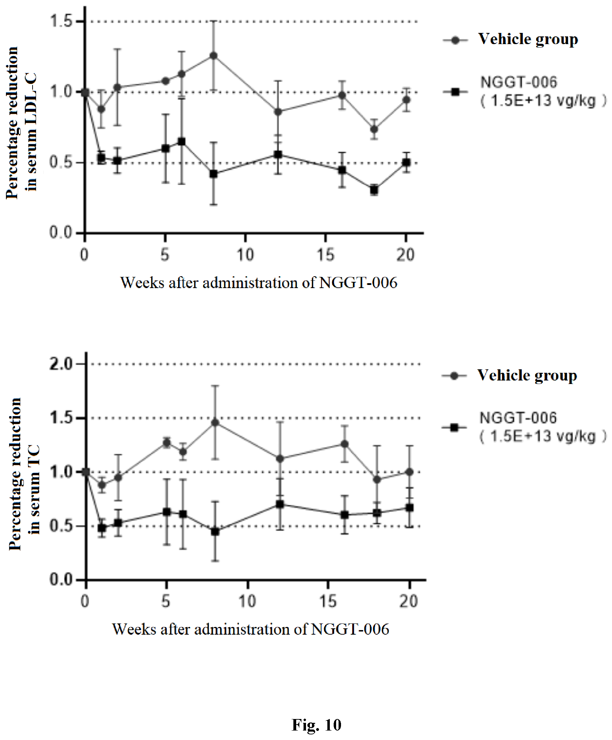

To investigate the lipid-lowering effect of NGGT-006 on LDLR heterozygous genotypes, in this example, 30-week-old female LDLR^{+/-} golden hamsters fed with natural diet ( SPF maintenance feed for mice and rats, purchased from Pizhou Xiaohe Technology Development Co., Ltd.) were randomly divided into experimental and control groups, with 5 in each group. At week 0, a single dose was administered via external jugular vein injection, with the experimental group receiving 1.5E+13 vg/kg of NGGT-006 virus (prepared in Example 1) and the control group receiving an equal volume of vehicle. The LDL-C and TC levels in the serum of the golden hamsters were measured at different time points after injection, and the results are as shown in Figure 10.

As shown in the figure, under normal dietary conditions, heterozygous golden hamsters exhibited slightly elevated LDL -C and TC levels in the serum (control group). In contrast, one week after administration with NGGT-006, the LDL-C and TC levels in the serum were significantly reduced (to approximately 50%), and this reduction was maintained over a long-term (for at least 20 weeks). These results indicated that the lipid-lowering effect of NGGT-006 was effective not only in homozygous genotypes lacking endogenous LDLR expression, but also in LDLR^{+/-} heterozygous genotypes with a certain amount of endogenous LDLR gene expression, suggesting that gene therapy based on the optimized sequence disclosed in this disclosure has the potential for application in both LDLR^{-/-} homozygous and LDLR^{+/-} heterozygous populations.

The above embodiments are merely specific implementations of the present disclosure, used to illustrate the technical solutions of the present disclosure, and are not intended to limit it. The scope of protection of the present disclosure is not limited thereto. Although the present disclosure has been described in detail with reference to the foregoing embodiments, those skilled in the art should understand that any person skilled in the art can still modify or easily conceive of changes to the technical solutions described in the foregoing embodiments within the scope of the technology disclosed in the present disclosure, or make equivalent substitutions for some of the technical features; and these modifications, changes, or substitutions do not cause the essence of the corresponding technical solutions to deviate from the spirit and scope of the technical solutions of the embodiments of the present invention, and should all be covered within the scope of protection of the present disclosure.

## Claims

1. A nucleic acid molecule encoding a human low-density lipoprotein receptor, wherein the nucleic acid molecule comprises the nucleotide sequence as shown in any one of SEQ ID NOs: 2-18; preferably, the nucleotide sequence is as shown in SEQ ID NO: 3, SEQ ID NO: 4, SEQ ID NO: 5, SEQ ID NO: 11, SEQ ID NO: 12, or SEQ ID NO: 16.

2. A transgene expression cassette comprising the nucleic acid molecule according to claim 1.

3. The transgene expression cassette according to claim 2, wherein the transgene expression cassette further comprises a promoter, an intron, and a terminator, and wherein the 3' end of the intron is operably linked to the 5' end of the nucleic acid molecule, the 3' end of the promoter is operably linked to the 5' end of the intron, and the 3' end of the nucleic acid molecule is operably linked to the 5' end of the terminator.

4. The transgene expression cassette according to claim 3, wherein the promoter is selected from the group consisting of a SerpinA1 gene promoter, a SerpinG1 gene promoter, an ALB gene promoter, an HLP gene promoter, or an LP gene promoter; preferably, the promoter is the SerpinA1 gene promoter; more preferably, the promoter comprises the nucleotide sequence as shown in SEQ ID NO: 21; and/or
the intron is a truncated SerpinA1 intron, preferably the truncated SerpinA1 intron comprises the nucleotide sequence as shown in SEQ ID NO: 22; and/or
the terminator is selected from the group consisting of BGH polyA, hGH polyA, or GH polyA, preferably BGH polyA, more preferably the BGH polyA comprises the nucleotide sequence as shown in SEQ ID NO: 23.

5. The transgene expression cassette according to any one of claims 3-4, further comprising a 5' ITR, a 3' ITR, and an enhancer, wherein the 5' ITR is located at the 5' end of the transgene expression cassette, the 3' ITR is located at the 3' end of the transgene expression cassette, and the 3' end of the enhancer is operably linked to the 5' end of the promoter.

6. The transgene expression cassette according to claim 5, wherein the enhancer is an ApoE HCR enhancer, preferably the ApoE HCR enhancer comprises the nucleotide sequence as shown in SEQ ID NO: 20;
further preferably, the transgene expression cassette comprises the nucleotide sequence as shown in SEQ ID NO: 32.

7. A recombinant adeno-associated virus comprising an AAV capsid and the nucleic acid molecule according to claim 1 or the transgene expression cassette according to any one of claims 2-6; preferably, the AAV is selected from the group consisting of AAV1, AAV2, AAV3, AAV4, AAV5, AAV6, AAV7, AAV8, AAV9, or rhAAV10; more preferably, the AAV is AAV8.

8. A composition comprising the nucleic acid molecule according to claim 1, the transgene expression cassette according to any one of claims 2-6, or the recombinant adeno-associated virus according to claim 7; preferably, the composition is a pharmaceutical composition, more preferably the pharmaceutical composition further comprises a pharmaceutically acceptable carrier and/or excipient.

9. Use of the nucleic acid molecule according to claim 1, the transgene expression cassette according to any one of claims 2-6, the recombinant adeno-associated virus according to claim 7, or the composition according to claim 8 in the preparation of a medicament for treating hypercholesterolemia, preferably familial hypercholesterolemia.

10. The use according to claim 9, wherein the familial hypercholesterolemia comprises homozygous familial hypercholesterolemia and heterozygous familial hypercholesterolemia.
